# EUROPEAN PATENT SPECIFICATION

(11) **EP 4 558 070 B1**
(45) Date of publication and mention of the grant of the patent: **21.10.2026**
(21) Application number: 22753897.2
(22) Date of filing: 20.07.2022
(51) Int. Cl.: A61B 17/3207, A61B 18/24, A61B 18/14, A61B 17/32, A61B 17/00, A61B 17/11

(54) **CATHETERS FOR ENDOVASCULAR TREATMENT OF A BLOOD VESSEL**
KATHETER ZUR ENDOVASKULÄREN BEHANDLUNG EINES BLUTGEFÄSSES
CATHÉTERS POUR TRAITEMENT ENDOVASCULAIRE D'UN VAISSEAU SANGUIN

(43) Date of publication of application: 28.05.2025
(73) Proprietor: Bard Peripheral Vascular, Inc., Franklin Lakes, NJ 07417 (US)
(72) Inventor: AKERELE-ALE, Oladipo Peter, Franklin Lakes, New Jersey (US); MOLL, Andrew, Franklin Lakes, New Jersey 07417 (US); ROMINGER, Kristin, Franklin Lakes, New Jersey 07417 (US); PALMER, Olivia, Franklin Lakes, New Jersey 07417 (US); SIMPSON, Breanna, Franklin Lakes, New Jersey 07417 (US); PALMER, Alexander, Franklin Lakes, New Jersey 07417 (US)
(74) Representative: Hoffmann Eitle
(86) International application number: PCT/US2022/037719
(87) International publication number: WO 2024/019717

(56) References cited:
- US-A1- 2003 181 938
- US-A1- 2017 007 311
- US-B1- 10 292 708
- US-B2- 9 474 562

## Description

### TECHNICAL FIELD

The present disclosure relates to catheters, devices, and methods for endovascular treatment of a blood vessel, and more particularly, catheters, devices, and methods that include a distal tip assembly having selectable size options for forming a fistula or providing other endovascular treatment.

### BACKGROUND

Endovascular treatments of a blood vessel may include fistula formation. A fistula is generally a passageway formed between two internal organs (e.g., blood vessels or other bodily organs). Forming a fistula between two blood vessels can have one or more beneficial functions. For example, the formation of a fistula between an artery and a vein may provide access to the vasculature for hemodialysis or hypertension patients. When a fistula is formed, minimum blood flow is required through the fistula, but at the same time, too much blood flow may lead to cardiac complications.

US 2003/181938 A1, US 10292708 B1 and US 9474562 B2 disclose catheters having distal fistula forming elements.

### SUMMARY

One challenging aspect of selecting a fistula size for forming a fistula (endovascular treatment) between blood vessels or though other body vessels, is selecting a fistula size during a procedure. A device initially advanced through a blood vessel may be later taken out of the blood vessel and changed out for another device to form a fistula in a different size. Embodiments of the present disclosure are directed to catheters, devices, and methods for endovascular treatments of a blood vessel that provide selectable size options for forming a fistula or providing other endovascular treatment as will be described in greater detail below.

The invention is defined by claim 1. Further embodiments of the invention are defined by the dependent claims.

In one embodiment, a catheter for endovascular treatment of a blood vessel is provided. The catheter includes a catheter body including a distal end and a distal tip assembly movably connected to the catheter body at the distal end of the catheter body. The distal tip assembly includes a plurality of tips that nest together and of which each comprises a fistula forming element and a support shaft. At least one of the plurality of tips is independently movable relative to an adjacent one of the plurality of tips between a retracted position and an extended position to provide a treatment region therebetween.

In another embodiment, a method of using a catheter is provided. The method includes holding a handle of the catheter for using the catheter. The catheter includes a catheter body extending distally from the handle, and a distal tip assembly movably connected to the catheter body at a distal end of the catheter body. The distal tip assembly includes a plurality of tips that nest together each comprising a fistula forming element and a support shaft. At least one of the plurality of tips is independently movable relative to an adjacent one of the plurality of tips between a retracted position and an extended position to provide a treatment region therebetween. The method further includes moving the at least one of the plurality of tips to the extended position and moving the at least one of the plurality of tips toward the retracted position.

In yet another embodiment, a surgical device is provided. The surgical device includes a catheter for endovascular treatment of a blood vessel. The catheter includes a catheter body and a distal tip assembly movably connected to the catheter body at a distal end of the catheter body. The distal tip assembly includes a first tip and a second tip adjacent the first tip. The first tip is connected to a support shaft and includes a fistula forming element. The first tip is movable between extended and retracted positions relative to the second tip to move the fistula forming element toward and away from the second tip.

These and additional features provided by the embodiments described herein will be more fully understood in view of the following detailed description, in conjunction with the drawings.

### BRIEF DESCRIPTION OF THE DRAWINGS

The embodiments set forth in the drawings are illustrative and exemplary in nature and not intended to limit the subject matter defined by the claims. The following detailed description of the illustrative embodiments can be understood when read in conjunction with the following drawings, where like structure is indicated with like reference numerals and in which:
FIG. 1A schematically depicts a catheter for endovascular treatment of a blood vessel having a distal tip assembly including a plurality of tips, where the tips are in a retracted position according to one or more embodiments shown and described herein;
FIG. 1B schematically depicts a catheter for endovascular treatment of a blood vessel, where a distal tip is at an extended position, according to one or more embodiments shown and described herein;
FIG. 1C schematically depicts a catheter for endovascular treatment of a blood vessel, where a middle tip is at an extended position, according to one or more embodiments shown and described herein;
FIG. 1D schematically depicts a catheter for endovascular treatment of a blood vessel, where a proximal tip is at an extended position, according to one or more embodiments shown and described herein;
FIG. 2A schematically depicts a cross sectional view of one embodiment of the catheter of FIG. 1A taken along section line 2-2, according to one or more embodiments shown and described herein;
FIG. 2B schematically depicts a cross sectional view of another embodiment of the catheter of FIG. 1A taken along section line 2-2, according to one or more embodiments shown and described herein;
FIG. 3A schematically depicts the catheter advanced through blood vessels, according to one or more embodiments shown and described herein;
FIG. 3B schematically depicts the middle tip of the catheter extended in the blood vessels, according to one or more embodiments shown and described herein;
FIG. 3C schematically depicts the middle tip of the catheter moved back to form a fistula between the blood vessels, according to one or more embodiments shown and described herein;
FIG. 3D schematically depicts the middle tip of the catheter retracted in the blood vessels, according to one or more embodiments shown and described herein;
FIG. 3E schematically depicts a fistula formed between the blood vessels and the catheter removed from the blood vessels, according to one or more embodiments shown and described herein;
FIG. 4A schematically depicts the catheter forming a fistula between blood vessels with the distal tip, according to one or more embodiments shown and described herein;
FIG. 4B schematically depicts the catheter forming a fistula between blood vessels with the middle tip, according to one or more embodiments shown and described herein; and
FIG. 4C schematically depicts the catheter forming a fistula between blood vessels with the proximal tip, according to one or more embodiments shown and described herein.

Reference will now be made in greater detail to various embodiments of the present disclosure, some embodiments of which are illustrated in the accompanying drawings. Whenever possible, the same reference numerals will be used throughout the drawings to refer to the same or similar parts.

### DETAILED DESCRIPTION

Embodiments described herein are directed to devices, systems, and methods for endovascular treatment of a blood vessel such as, but not limited to forming a fistula, bypass procedures, etc. For example, a catheter may be placed in each of two adjacent blood vessels to form a fistula therebetween with the catheter. When a fistula is formed, minimum blood flow is required through the fistula, but at the same time, too much blood flow may lead to cardiac complications. One of the factors that affects a blood flow level is a fistula size. It may therefore be useful to have devices providing selectable treatment region size options for forming a fistula or providing other endovascular treatment. Embodiments of the present disclosure provide selectable treatment region size options for forming a fistula or providing other endovascular treatment. For example, in some embodiments, a catheter for endovascular treatment of a blood vessel according to the present disclosure includes a catheter body, and a distal tip assembly movably connected to the catheter body at a distal end of the catheter body. The distal tip assembly includes a plurality of tips each including a fistula forming element and a support shaft. The plurality of tips are independently movable between an extended position and a retracted position, wherein adjacent tips define a treatment region. These and additional features and benefits will be described in greater detail herein.

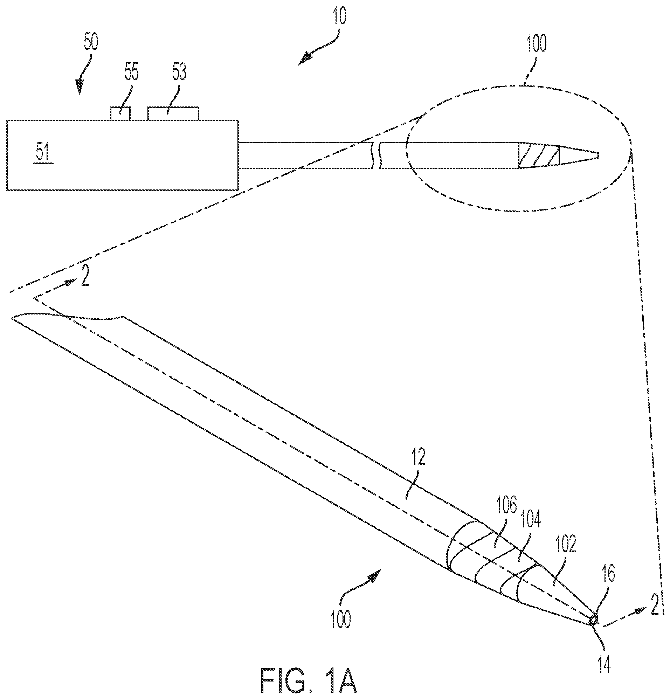

Referring to FIGS. 1A-1D, a surgical device including a catheter 10 for providing endovascular treatment of a blood vessel, such as a fistula formation, and a handle 50 is schematically depicted. The catheter 10 may have a distal tip assembly 100 movably connected to the catheter body 12. The catheter 10 may additionally comprise one or more lumens or passageways extending at least partially along or through the catheter, and may be used to pass one or more guidewires, one or more drugs or fluids (e.g., contrast agents, perfusion fluids), combinations thereof, or the like at least partially along or through the catheter 10. The distal end portion of the catheter may be configured to aid in advancement of the catheter and/or to be atraumatic. In some variations, the distal end portion may comprise one or more rapid exchange portions or other lumens for advancement of the catheter over a guidewire. In still other variations, the distal end portion may have a guidewire attached to or otherwise integrally formed with the catheter 10.

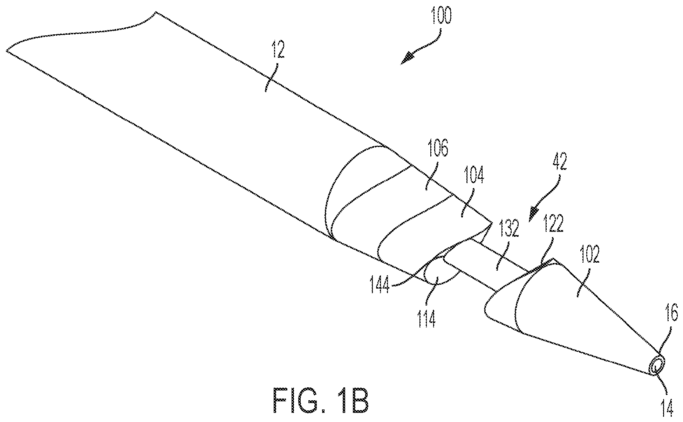

Referring now to FIG. 1A, the distal tip assembly 100 may have tips including a distal tip 102, a middle tip 104, and a proximal tip 106. The distal tip assembly 100 is coupled to the catheter body 12 via support shafts. Each of the distal tip 102, the middle tip 104, and the proximal tip 106 has a respective support shaft 132, 134 and 136 (FIGS. 1B-1D) that is disposed in the catheter body 12. The catheter 10 may have a lumen 14 in which a guidewire may be advanced through to assist positioning of the catheter 10 in a blood vessel. The catheter 10 may have any suitable diameter for intravascular use, such as, for example, about 4 French, about 5.7 French, about 6.1 French, about 7 French, about 8.3 French, between about 4 French and about 9 French, between about 4 French and about 7 French, between about 4 French and about 6 French, or the like. The catheter 10 may further comprise elements to aid in visualization and/or alignment of catheter 10 as described in more detail herein. French = 1/3 mm.

The catheter 10 may be held by a handle 50 that a user may hold to operate the catheter 10. The handle 50 may include a body 51 with an activation switch 55 and a size selector 53. The activation switch 55 may activate a fistula forming element of the distal tip assembly. The activation switch 55 may be a push button that is pushed to activate the fistula forming element and released to deactivate the fistula forming element. The size selector 53 may be used to selectably extend and retract the tips of the distal tip assembly 100. The size selector 53 may be a thumb drive that a user can rotate to manually extend and retract individual tips. For example, the size selector 53 may have a plurality of thumb drives correspond to each tip 102, 104 and 106. The size selector 53 may be an automatic selector coupled to a user interface and motors to automatically extend and retract the tips based on input from the user. While a push button and thumb drive is shown and described, any suitable user input may be provided. For example, a touch screen may be provided that includes selectable input areas that can be actuated to send control signals to a controller to control one or more motors for extending and retracting one or more of the plurality of tips 102, 104 and 106.

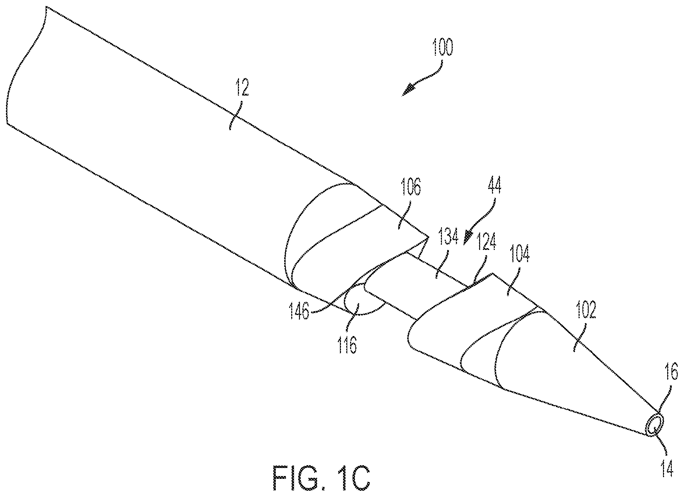

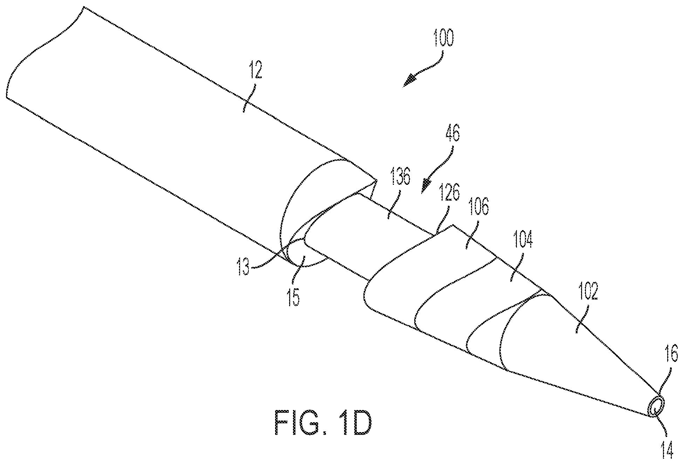

The distal tip assembly 100 is in a retracted position in FIG. 1A. When the distal tip assembly 100 is retracted, the distal tip 102, the middle tip 104, and the proximal tip 106 respectively are in a retracted position. The distal tip assembly 100 may be tapered toward a distal end 16 of the catheter 10. The outer surfaces of distal tip 102, the middle tip 104, and the proximal tip 106 may be flush with each other to provide a smooth and continuous outer surface. Each of the distal tip 102, the middle tip 104, and the proximal tip 106 may define treatment regions 42, 44, and 46 there between. Treatment regions 42, 44, and 46, as used herein, refer to spaces between adjacent tips when the tips are extended. For example, the treatment region 42 is defined between the distal tip 102 and the middle tip 104 when the distal tip 102 is extended (FIG. 1B). The treatment region 44 is defined between the middle tip 104 and the proximal tip 106 when the middle tip 104 is extended (FIG. 1C). The treatment region 46 is defined between the proximal tip 106 and the catheter body 12 when the proximal tip 106 is extended (FIG. 1D). The treatment regions 42, 44, and 46 may have different sizes (e.g., diameters). For example, the outermost diameter of the distal tip 102 may be smaller than the outermost diameters of the middle tip 104 and the proximal tip 106 due to the overall tapering shape of the distal tip assembly 100. Additionally, the outermost diameter of the middle tip 104 may be smaller than the outermost diameter of the proximal tip 106. The distal end 16 may also be part of the tapered configuration of the distal tip assembly 100. In some embodiments, the distal end 16 may have a reduced size than the catheter body 12. For example, the distal end 16 may have smaller diameter than the catheter body 12 or the distal end 16 may have a diagonal surface such that the size of the distal end 16 is reduced toward the distal end 16. In some embodiments, the distal tip assembly 100 has an outer surface continuously and/or gradually tapered toward the distal end 16 of the catheter 10. The tapered distal tip assembly 100 may easily advance through the blood vessel.

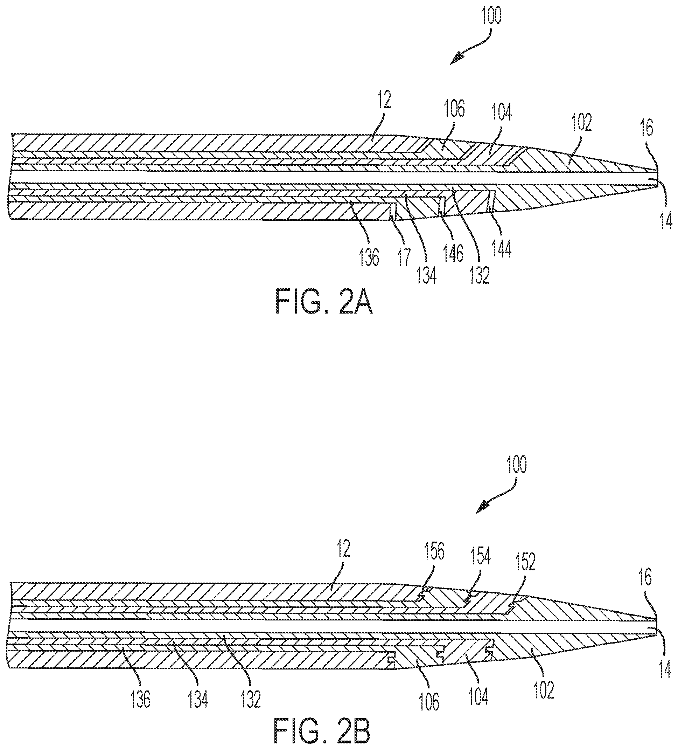

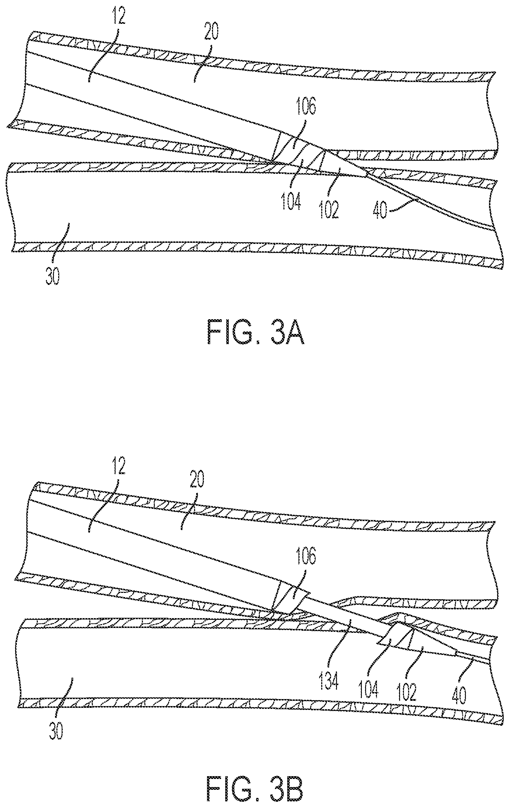

Referring to FIG. 1B, the distal tip 102 is in an extended position. When the distal tip 102 is in the extended position, the middle tip 104 and the proximal tip 106 may respectively stay in a retracted position. In this regard, each of the distal tips 102, 104 and 106 have their own extended and retracted position. The distal tip 102 may have the shaft 132 that extends through the catheter body 12, the proximal tip 106, and the middle tip 104. The shaft 132 is moved or slid out from an inner bore 144 of the middle tip 104. The configuration of the shaft 132 will be discussed later in detail with reference to FIGS. 2A-2B and 3A-3E.

The distal tip 102 may include a proximal surface 122 that faces a distal surface 114 of the middle tip 104. In some embodiments, the proximal surface 122 and the distal surface 114 may be flush with each other with the distal tip 102 in its retracted position. When the distal tip 102 is in the retracted position, the proximal surface 122 may nest with the distal surface 114. For example, the shape and size of the proximal surface 122 and the distal surface 114 may correspond such that the two surfaces 122 and 114 interlock and provide a continuous, smooth interface between the surfaces 122 and 114. Although the proximal surface 122 and the distal surface 114 are illustrated as wavy or undulating surfaces, they may be flat surfaces that are either angled or perpendicular to the shaft 132. The shape and angle of the proximal surface 122 and the distal surface 114 may be modified depending on a particular anatomy of a site to be treated or a size or a shape of a fistula to be formed. The proximal surface 122 and the distal surface 114 may be textured to provide grip to hold tissue to be treated between the proximal surface 122 and the distal surface 114.

In some embodiments, as discussed above, the space between the distal tip 102 and the middle tip 104 may define a treatment region 42. The treatment region 42 is the space where tissue is disposed when being treated. For example, tissue may be held or pinched in the treatment region 42 between the distal tip 102 and the middle tip 104 during operation. The tissue may be treated by a fistula forming element, discussed in greater detail below with reference to FIGS. 2A, 2B and 3A-3E. The fistula forming element may deliver energy to the treatment region to form a fistula. The fistula forming element may be disposed on one or both of the proximal surface 122 and the distal surface 114.

Referring to FIG. 1C, the middle tip 104 is in an extended position. When the middle tip 104 is in the extended position, the proximal tip 106 may stay in a retracted position. In this regard, each of the distal tips 102, 104, and 106 have their own extended and retracted position. The middle tip 104 may have a shaft 134 that extends through the catheter body 12 and the proximal tip 106. The shaft 134 is moved or slid out from an inner bore 146 of the proximal tip 106. When the middle tip 104 is in the extended position, the distal tip 102 also extended or moved away from the proximal tip 106 such that the middle tip 104 and the distal tip 102 are extended together. In other words, the proximal tip 106 stays in the retracted position when the middle tip 104 and the distal tip 102 are extended. The shaft 132 of the distal tip 102 may stay disposed inside of the middle tip 104 when the middle tip 104 is in the extended position. The shaft 134 has a greater outer diameter than the shaft 132 of the distal tip 102. The configuration of the shaft 134 will be discussed later in detail with reference to FIGS. 2A-2B and 3A-3E.

The middle tip 104 may include a proximal surface 124 that faces a distal surface 116 of the proximal tip 106. In some embodiments, the proximal surface 124 and the distal surface 116 may flush with each other with the middle tip 103 in its retracted position. When the middle tip 104 is in the retracted position, the proximal surface 124 may nest with the distal surface 116. For example, the shape and size of the proximal surface 124 and the distal surface 116 may correspond such that the two surfaces 124 and 116 interlock and provide a continuous, smooth interface between the surfaces 124 and 116. Although the proximal surface 124 and the distal surface 116 are illustrated as wavy or undulating surfaces, they may be flat surfaces that are either angled or perpendicular to the shaft 134. The shape and the angle of the proximal surface 124 and the distal surface 116 may be modified depending on a particular anatomy of a site to be treated or a size or a shape of a fistula to be formed. The proximal surface 124 and the distal surface 116 may be textured to provide grip to hold tissue to be treated between the proximal surface 124 and the distal surface 116.

In some embodiments, the middle tip 104 and the proximal tip 106 may define a treatment region 44. The treatment region 44 is the space where tissue is disposed when being treated. For example, tissue may be held or pinched in the treatment region 44 between the middle tip 104 and the proximal tip 106 during operation. The tissue may be treated by a fistula forming element, discussed in greater detail below with reference to FIGS. 2A, 2B and 3A-3E. The fistula forming element may deliver energy to the treatment region to form a fistula. The fistula forming element may be disposed on one or both of the proximal surface 124 and the distal surface 116.

Referring to FIG. 1D, the proximal tip 106 is in an extended position. When the proximal tip 106 is in the extended position, the middle tip 104 and the distal tip 102 are also extended or moved away from the catheter body 12 such that the proximal tip 106, the middle tip 104, and the distal tip 102 are moved together. In other words, the proximal tip 106, the middle tip 104, and the distal tip 102 are extended together when the proximal tip 106 is in the extended position. The proximal tip 106 may have a shaft 136 that extended through the catheter body 12. The shaft 136 is moved or slid out from an inner bore 13 of the catheter body 12. The shaft 132 of the distal tip 102 may stay disposed inside of the middle tip 104, and the shaft 134 of the middle tip 104 may stay disposed inside of the proximal tip 106, when the proximal tip 106 is in the extended position. The shaft 136 has a greater outer diameter than the shaft 134 of the middle tip 104 and the shaft 132 of the distal tip 102. The configuration of the shaft 136 will be discussed later in detail with reference to FIGS. 2A-2B and 3A-3E.

The proximal tip 106 may include a proximal surface 126 that faces a distal surface 15 of the catheter body 12. In some embodiments, the proximal surface 126 and the distal surface 15 may flush with each other with the proximal tip 106 in its retracted position. When the proximal tip 106 is in the retracted position, the proximal surface 126 may nest with the distal surface 15. For example, the shape and size of the proximal surface 126 and the distal surface 15 may correspond such that the two surfaces 126 and 15 interlock and provide a continuous, smooth interface between the surfaces 126 and 15. Although the proximal surface 126 and the distal surface 15 are illustrated as wavy or undulating surfaces, they may be flat surfaces that are either angled or perpendicular to the shaft 136. The shape and the angle of the proximal surface 126 and the distal surface 15 may be modified depending on a particular anatomy of a site to be treated or a size or a shape of a fistula to be formed. The proximal surface 126 and the distal surface 15 may be textured to provide grip to hold tissue to be treated between the proximal surface 126 and the distal surface 15.

In some embodiments, the proximal tip 106 and the catheter body 12 may define a treatment region 46. The treatment region 46 is the space where tissue is disposed when being treated. For example, tissue may be held or pinched in the treatment region 46 between the proximal tip 106 and the catheter body 12 during operation. The tissue may be treated by a fistula forming element, discussed in greater detail below with reference to FIGS. 2A, 2B and 3A-3E. The fistula forming element may deliver energy to the treatment region 46 to form a fistula. The fistula forming element may be disposed on one or both of the proximal surface 126 and the distal surface 15.

In some embodiments, referring to FIG. 2A, a cross sectional view of the catheter 10 taken along section line 2-2 of FIG. 1 is shown. The distal tip assembly 100 is in a retracted position, such that the distal tip 102, the middle tip 104, and the proximal tip 106, respectively, are in a retracted position. The shafts 132, 134, and 136 are nested along at least a part of their lengths. The shaft 132 of the distal tip 102 is disposed in the shaft 134 of the middle tip 104, and the shaft 134 of the middle tip 104 is disposed in the shaft 136 of the proximal tip 106. In the illustrated example, all of the shafts 132, 134 and 136 of the proximal tip 106 extend through the catheter body 12 and may be operatively connected to the size selector 53. Each shaft 132, 134, and 136 may be individually movable so that each of the distal tip 102, the middle tip 104, and the proximal tip 106 may individually slide in a longitudinal direction between an extended position and a retracted position as shown in FIGS. 1A-1D and described above with reference to FIG. 1A. In some embodiments, the shafts 132, 134, and 136 are disposed with a gap there between to allow the shafts 132, 134, and 136 to slide. The gap could be modified to add or reduce more clearance.

As discussed above, the fistula forming element may be disposed on the one or both of each of the proximal surface and the distal surface of respective tips. For example, the distal surface 114 of the middle tip 104 may include a fistula forming element 144. Similarly, the distal surface 116 of the proximal tip 106 may include a fistula forming element 146. Further, the distal surface 15 of the catheter body 12 may include a fistula forming element 17. The fistula forming elements 144, 146, and 17 may be coupled to one or more generators for supplying energy to the fistula forming elements 144, 146, and 17. The fistula forming elements 144, 146, and 17 may utilize, for example, but not limited to, thermal energy, radio frequency (RF) energy, or laser energy to ablate or weld tissue.

As discussed above in FIG. 1, the activation switch 55 may activate the fistula forming elements 17, 144, and 146 independently or together using a power source that is electrically connected to the activation switch 55 and the fistula forming elements 17, 144 and 146. A user of the catheter 10 may push the activation switch 55 to activate or deactivate a fistula forming element 144, 146 and 17 corresponding to a retracted tip. For example, when the distal tip 102 is retracted against tissue, pushing the activation switch 55 may be used to activate the fistula forming element 144 to cut, ablate, or weld the tissue. Similarly, when the middle tip 104 and/or the proximal tip 106 are retracted, pushing the activation switch 55 may activate the fistula forming element 146 and/or 17 to cut, ablate, or weld tissue.

The fistula forming elements 144, 146, and 13 may cover the entire distal surfaces 114, 116, and 15. For example, each of the fistula forming elements 144, 146, and 17 may include an electrode covered with a heat conductive material to spread heat. The heat conductive material may include, but not limited to, metals including aluminum, stainless steel, and aluminum nitride, or ceramic materials. In some variants, an exposed surface of the electrode may comprise a porous coating that allows conduction of current thereto or therefrom while preventing the electrode from direct contact with an opposing facing surface. The electrode may be made from any suitable material or combination of materials. In some variations the electrode may comprise one or more refractory metals. For example, an electrode may comprise tungsten, molybdenum, niobium, tantalum, rhenium, combinations or alloys thereof.

The outer most surface of the fistula forming elements 144, 146, and 17 may have any shape or size suitable for joining and/or ablating tissue. In some embodiments, the fistula forming element may be disposed only partially on the one or both of the proximal surface and the distal surface of respective tips. For example, the fistula forming elements 144, 146 and 17 may include a wire or a plate that is disposed on the surface of one or both of the proximal surface and the distal surface. The fistula forming elements 144, 146, and 17 may be disposed in a cavity formed on one or both of the proximal surface and the distal surface. The fistula forming elements 144, 146, and 17 may be surrounded by a material that may insulate the fistula forming elements 144, 146, and 17 from surrounding tissue or other portions of the catheter 10, which may protect or shield the other portions of the catheter. For example, thermal insulation provided by the material may protect other catheter components from heat that may be generated by the electrode. Additionally or alternatively, electrical insulation provided by the material may help minimize current loss to other parts of the catheter 10 or surrounding tissue. The material may be made of any heat and/or electrically resistant materials. Examples of suitable materials include, but are not limited to, ceramic materials, parylene, one or more polymeric resins (e.g., polyetherimide, polyetheretherketone, one or more phenolic resins, or the like), silica, one or more metal oxides (e.g., aluminum oxide), combinations thereof, or the like.

The lumen 14 is extended along the catheter 10 to allow a guidewire to be advanced there through. The distal end 16 may be sharp enough to puncture tissue. For example, no additional device may be needed for initial cutting of tissue to advance the catheter 10 through the tissue when the distal end 16 is sharp enough to puncture tissue. On the other hand, the distal end 16 may be blunt so that may not puncture or scratch tissue. In that case, an additional device may be used for initial cutting of tissue to advance the catheter 10 through the tissue.

In other embodiments, referring to FIG. 2B is a cross sectional view of the catheter 10 taken along section line 2-2. The catheter 10 in the embodiments illustrated in FIG. 2B is similar to FIG. 2A, except there are protrusions 152, 154 and 156 disposed between the tips including the distal tip 102, the middle tip 104, and the proximal tip 106. The protrusions 152, 154 and 156 may have a ring shape that surrounds the shaft of the respective tips. For example, the distal tip 102 has the protrusion 152 that is extended along the proximal surface 122 of the distal tip 102 such that the protrusion 152 has a ring shape viewed from proximal direction of the catheter 10. Similarly, the middle tip 104 has the protrusion 154 that is extended along the proximal surface 124 of the middle tip 104 such that the protrusion 154 has a ring shape viewed from proximal direction of the catheter 10. The proximal tip 106 has the protrusion 156 that is extended along the proximal surface 126 of the proximal tip 106 such that the protrusion 156 has a ring shape viewed from proximal direction of the catheter 10. Each distal surface 114, 116, and 15 has a recess (also at 152, 154 and 156) that receives the protrusions 152, 154, and 156, respectively. The protrusions 152, 154, and 156 may have different cross-sectional shapes and sizes. For example, the protrusions 152, 154, and 156 may have cross-sectional shapes that are a square, rectangle, or round. In some embodiments, the protrusions 152, 154, and 156 may not extend continuously and/or entirely around the respective shafts 132, 134, and 136. In some embodiments, the fistula forming elements may be disposed on the protrusions 152, 154, and 156. The size of shape of the 152, 154, and 156 may be modified depending on the location and size of the fistula forming elements.

In some embodiments, referring to FIGS. 3A to 3E, the catheter 10 is advanced through two adjacent blood vessels 20 and 30 to perform treatment. For example, the catheter 10 may form a fistula between the two blood vessels 20 and 30.

Referring to FIG. 3A, the catheter 10 may be advanced through the blood vessel 20 along a guidewire 40 loaded into the lumen 14. The blood vessels 20 and 30 may be pierced by the distal end 16. In some embodiments, the blood vessels 20 and 30 may be pierced by a needle, which is inserted along the guidewire 40 and withdrawn after piercing the blood vessels 20 and 30. Once the blood vessels 20 and 30 are pierced, the catheter 10 may further be advanced through openings between the blood vessels 20 and 30. The tapered outer surface of the distal tip assembly 100 may further enlarge the openings depending on a desired fistula size.

The distal tip assembly 100 may provide multiple selectable size options. For example, the distal tip assembly 100 provides three size options that are provided by the distal tip 102, the middle tip 104, and the proximal tip 106. The outer diameter may increase from the distal tip 102, to the middle tip 104, and to the proximal tip 106. Also, the diameters of the shafts may increase from the shaft 132, to the shaft 134, and to the shaft 136. Therefore, the size of a fistula formed between the tips 102, 104 and 106 may increase from the distal tip 102, to the middle tip 104, and to the proximal tip 106, in that order. The size may be chosen based on factors including, but not limited to, blood flow, blood pressure, or the like. Further, the size choice may depend on a balance between cardiovascular impacts and maturation rates. Practitioners may choose from the multiple size options based on his or her own experiences.

In some embodiments, the distal tip assembly 100 may provide two size options. For example, the distal tip assembly 100 may have two tips instead of three tips. In other embodiments, the distal tip assembly 100 may provide four or more size options. For example, the distal tip assembly 100 may have four or more tips. Regardless of the numbers of the tips provided, the outer diameter of each of the tips may increase from the most distal tip to the most proximal tip, in that order. Similarly, the outer diameter of each of the shafts of the respective tips may increase from the most distal tip to the most proximal tip, in that order.

Referring to FIG. 3B, a selected tip may be extended to be advanced through the opening between the blood vessels 20 and 30. For example, the middle tip 104 may be extended through the opening. The vessel walls of the blood vessels 20 and 30 around the opening may be stretched while the middle tip 104 advances through the opening. The walls may be recovered back to a smaller size such that the walls surrounding the opening may fit around the shaft 134 of the middle tip 104. The distal surface 116 of the proximal tip 106 may prevent the proximal tip 106 to be advanced through the opening when the middle tip 104 is advanced through the opening. Further advancing of the catheter 10 may advance the distal surface 116 and push the vessel wall of the blood vessel 20 toward the blood vessel 30. Therefore, the distal surface 116 may assist the vessel wall of the blood vessel 20 to remain in close proximity to the vessel wall of the blood vessel 30. The distal surface 116 may be positioned flush with the vessel wall of the blood vessel 20.

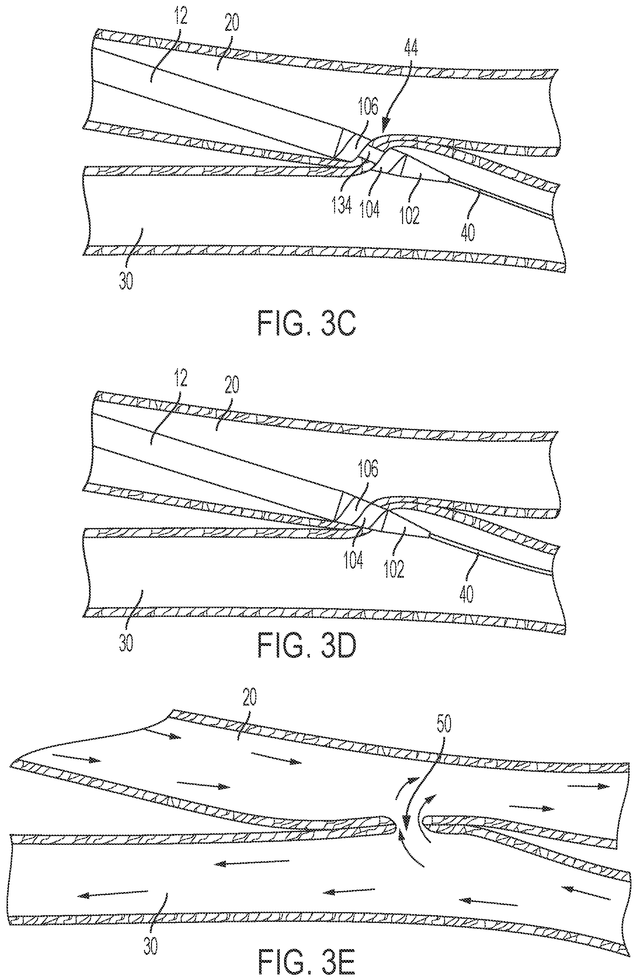

Referring to FIG. 3C, the selected tip may be retracted to hold or pinch the blood vessels 20 and 30. For example, the middle tip 104 may be moved closer to the proximal tip 106 by retracting the distal and middle tips 102 and 104 together relative to the proximal tip 106. The vessel walls of the blood vessels 20 and 30 may held or pinched between the proximal surface 124 of the middle tip 104 and the distal surface 116 of the proximal tip 106. A tension may be applied by a manual operation of the distal tip assembly by pulling the middle tip 104 back in a proximal direction. The tension may compress the vessel walls held between the middle tip 104 and the proximal tip 106. The tension may be maintained while treating the blood vessels. The angled proximal surface 124 and the distal surface 116 may reduce stress applied to the vessel walls of the blood vessels 20 and 30 from the tension.

Referring to FIG. 3D, when the vessel walls are held between the middle tip 104 and the proximal tip 106, energy may be delivered to the treatment region. As discussed above, the distal surface 116 may have the fistula forming element 146, which may be energized to deliver energy to the vessel walls disposed in the treatment region. For example, heat may be generated by the fistula forming element 146 to cut the vessel walls or to weld the vessel walls to form a fistula. The energy level may be maintained to weld to join the vessel walls together without cutting through the vessel walls. The energy level also may be adjusted based on characteristics of blood vessels. For example, the energy level may be adjusted based on the impedance of the vessel walls. If RF energy is utilized, a variety of RF energy profiles may be applied to the treating element. For example, different pulse or duty cycles may be utilized to reduce heat impact to adjacent tissues. After providing the treatment, the catheter 10 may be withdrawn from the blood vessels 20 and 30. The guidewire 40 may or may not be withdrawn depending on remaining procedures.

Referring to FIG. 3E, a fistula 50 is formed between the blood vessel 20 and the blood vessel 30. The fistula 50 creates a passage between the blood vessels 20 and 30 and blood flow may be established through the fistula 50. As illustrated, tissues surrounding the passage are welded, and the welded surface area may correspond to the surface area of the fistula forming element.

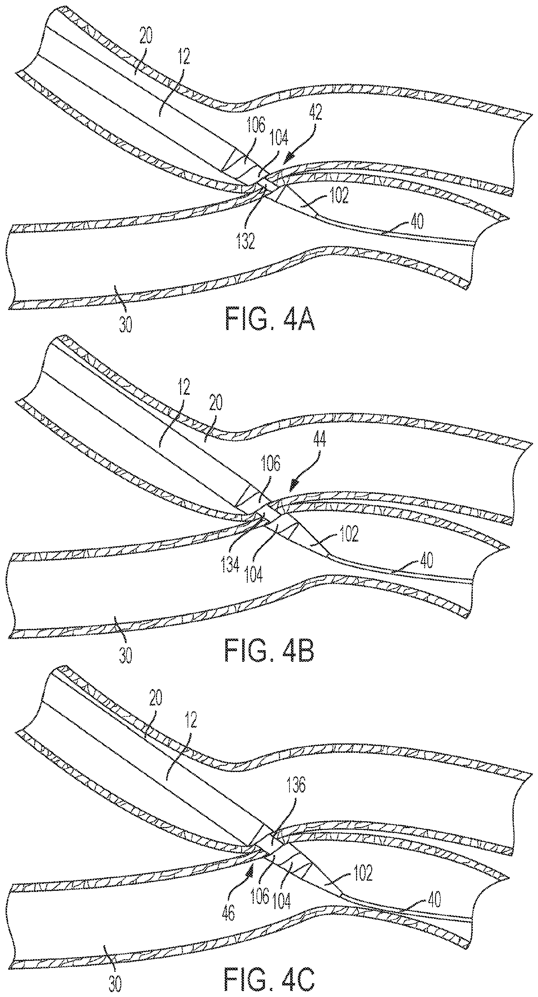

Referring to FIG. 4A-4B, illustrates different tips are utilized to treat the blood vessels 20 and 30. For example, referring to FIG. 4A, the distal tip 102 is extended to hold vessel walls of the blood vessels 20 and 30 between the distal tip 102 and the middle tip 104. Referring to FIG. 4B, the proximal tip 106 is extended to hold vessel walls of the blood vessels 20 and 30 between the proximal tip 102 and the catheter body 12. As illustrated in FIGS. 3C, 4A-4B, the distal tip 102, the middle tip 104, and the proximal tip 106 may form fistulas in different sizes. It may therefore be provided selectable size options for forming a fistula or providing other endovascular treatments.

In some examples, a surgical device may allow the formation of a fistula with a controlled diameter, thus improving the quality of the endovascular treatment and reducing the risks of a post-surgical complication.

In some examples, the shape and inner arrangement of the distal tip assembly allows for a more compact arrangement and an easier advancement through a blood vessel.

In some examples, a dedicated surface, optimally oriented for fistula formation allows for more reliable fistula formation by a surgical device.

In some examples, a complementary shape of surfaces of the tips improves the precision of the interlocking of the tips, thus allowing for a better continuity and smoothness of the interface of the distal tip assembly.

It is noted that the terms "substantially" and "about" may be utilized herein to represent the inherent degree of uncertainty that may be attributed to any quantitative comparison, value, measurement, or other representation. These terms are also utilized herein to represent the degree by which a quantitative representation may vary from a stated reference without resulting in a change in the basic function of the subject matter at issue.

The invention is defined by the claims.

## Claims

1. A catheter (10) for endovascular treatment of a blood vessel, comprising:
a catheter body (12) including a distal end; and
a distal tip assembly (100) movably connected to the catheter body (12) at the distal end of the catheter body (12), the distal tip assembly (100) including a plurality of tips (102, 104, 106) that nest together and of which each comprises a fistula forming element (17, 144, 146) and a support shaft (132, 134, 136), wherein
at least one of the plurality of tips (102, 104, 106) is independently movable relative to an adjacent one of the plurality of tips (102, 104, 106) between a retracted position and an extended position to provide a treatment region (42, 44, 46) therebetween.

2. The catheter of claim 1, wherein an outer diameter of the adjacent one of the plurality of tips (102, 104, 106) is larger than an outer diameter of the at least one of the plurality of tips (102, 104, 106).

3. The catheter of claim 1 or 2, wherein the support shaft (132, 134, 136) of the at least one of the plurality of tips (102, 104, 106) extends through the support shaft (132, 134, 136) of the another of the plurality of tips (102, 104, 106).

4. The catheter of any one of the preceding claims, wherein the at least one of the plurality of tips (102, 104, 106) is located distal of the adjacent one of the plurality of tips (102, 104, 106), the treatment region (42, 44, 46) located between a proximally-facing surface of the at least one of the plurality of tips (102, 104, 106) and a distally-facing surface of the adjacent one of the plurality of tips (102, 104, 106) with the at least one of the plurality of tips (102, 104, 106) in the extended position.

5. The catheter of any one of the preceding claims, wherein the plurality of tips (102, 104, 106) define at least two treatment regions (42, 44, 46) therebetween.

6. The catheter of claim 4 or 5, wherein the distally-facing surface and the proximally-facing surface have non-planar contours.

7. The catheter of any one of the preceding claims, wherein the at least one of the plurality of tips (102, 104, 106) is a distal tip (102) and the adjacent one of the plurality of tips (102, 104, 106) is a middle tip (104), the plurality of tips (102, 104, 106) further comprising a proximal tip (106) that is adjacent the middle tip (104).

8. The catheter of any one of claims 4-6, wherein the proximally-facing surface comprises an interlocking portion that interlocks with an interlocking portion of the distally-facing surface facing the proximally-facing surface.

9. The catheter of claim 8, wherein one of the interlocking portions is a protrusion (152, 154, 156) and the other one of the interlocking portions is a recess.

## Patentansprüche

1. Katheter (10) zur endovaskulären Behandlung eines Blutgefäßes, umfassend:
einen Katheterkörper (12), der ein distales Ende einschließt; und
eine distale Spitzenbaugruppe (100), die an dem distalen Ende des Katheterkörpers (12) beweglich mit dem Katheterkörper (12) verbunden ist, wobei die distale Spitzenbaugruppe (100) eine Vielzahl von Spitzen (102, 104, 106) einschließt, die ineinander verschachtelt sind und von denen jede ein Fistelbildungselement (17, 144, 146) und einen Stützschaft (132, 134, 136) umfasst, wobei
mindestens eine der Vielzahl von Spitzen (102, 104, 106) relativ zu einer benachbarten einen der Vielzahl von Spitzen (102, 104, 106) zwischen einer zurückgezogenen Position und einer ausgefahrenen Position unabhängig beweglich ist, um einen Behandlungsbereich (42, 44, 46) dazwischen bereitzustellen.

2. Katheter nach Anspruch 1, wobei ein Außendurchmesser der benachbarten einen der Vielzahl von Spitzen (102, 104, 106) größer als ein Außendurchmesser der mindestens einen der Vielzahl von Spitzen (102, 104, 106) ist.

3. Katheter nach Anspruch 1 oder 2, wobei sich der Stützschaft (132, 134, 136) der mindestens einen der Vielzahl von Spitzen (102, 104, 106) durch den Stützschaft (132, 134, 136) der anderen der Vielzahl von Spitzen (102, 104, 106) hindurch erstreckt.

4. Katheter nach einem der vorstehenden Ansprüche, wobei sich die mindestens eine der Vielzahl von Spitzen (102, 104, 106) distal zu der benachbarten einen der Vielzahl von Spitzen (102, 104, 106) befindet, wobei sich der Behandlungsbereich (42, 44, 46) zwischen einer proximal zugewandten Fläche der mindestens einen der Vielzahl von Spitzen (102, 104, 106) und einer distal zugewandten Fläche der benachbarten Spitze der Vielzahl von Spitzen (102, 104, 106) befindet, wenn sich die mindestens eine der Vielzahl von Spitzen (102, 104, 106) in der ausgefahrenen Position befindet.

5. Katheter nach einem der vorstehenden Ansprüche, wobei die Vielzahl von Spitzen (102, 104, 106) mindestens zwei Behandlungsbereiche (42, 44, 46) dazwischen definiert.

6. Katheter nach Anspruch 4 oder 5, wobei die distal zugewandte Fläche und die proximal zugewandte Fläche nicht-ebene Konturen aufweisen.

7. Katheter nach einem der vorstehenden Ansprüche, wobei die mindestens eine der Vielzahl von Spitzen (102, 104, 106) eine distale Spitze (102) ist und die benachbarte der Vielzahl von Spitzen (102, 104, 106) eine mittlere Spitze (104) ist, wobei die Vielzahl von Spitzen (102, 104, 106) weiter eine proximale Spitze (106) umfasst, die der mittleren Spitze (104) benachbart ist.

8. Katheter nach einem der Ansprüche 4-6, wobei die proximal zugewandte Fläche einen Verriegelungsabschnitt umfasst, der mit einem Verriegelungsabschnitt der distal zugewandten Fläche verriegelt, die der proximal zugewandten Fläche zugewandt ist.

9. Katheter nach Anspruch 8, wobei einer der Verriegelungsabschnitte ein Vorsprung (152, 154, 156) ist und der andere der Verriegelungsabschnitte eine Aussparung ist.

## Revendications

1. Cathéter (10) pour le traitement endovasculaire d'un vaisseau sanguin, comprenant :
un corps de cathéter (12) incluant une extrémité distale ; et
un ensemble pointe distale (100) relié de manière mobile au corps de cathéter (12) à l'extrémité distale du corps de cathéter (12), l'ensemble pointe distale (100) incluant une pluralité de pointes (102, 104, 106) qui s'emboîtent l'une dans l'autre et dont chacune comprend un élément (17, 144, 146) de formage de fistule et une tige de support (132, 134, 136), dans lequel
au moins une de la pluralité de pointes (102, 104, 106) est indépendamment mobile par rapport à une adjacente de la pluralité de pointes (102, 104, 106) entre une position rétractée et une position étendue pour fournir une zone de traitement (42, 44, 46) entre celles-ci.

2. Cathéter selon la revendication 1, dans lequel un diamètre externe de l'une adjacente de la pluralité de pointes (102, 104, 106) est plus grand qu'un diamètre externe de l'au moins une de la pluralité de pointes (102, 104, 106).

3. Cathéter selon la revendication 1 ou la revendication 2, dans lequel la tige de support (132, 134, 136) de l'au moins une de la pluralité de pointes (102, 104, 106) s'étend à travers la tige de support (132, 134, 136) de l'autre de la pluralité de pointes (102, 104, 106).

4. Cathéter selon l'une quelconque des revendications précédentes, dans lequel l'au moins une de la pluralité de pointes (102, 104, 106) est située distalement de l'une adjacente de la pluralité de pointes (102, 104, 106), la zone de traitement (42, 44, 46) étant située entre une surface orientée proximalement de l'au moins une de la pluralité de pointes (102, 104, 106) et une surface orientée distalement de l'une adjacente de la pluralité de pointes (102, 104, 106) avec l'au moins une de la pluralité de pointes (102, 104, 106) dans la position étendue.

5. Cathéter selon l'une quelconque des revendications précédentes, dans lequel la pluralité de pointes (102, 104, 106) définit au moins deux zones de traitement (42, 44, 46) entre celles-ci.

6. Cathéter selon la revendication 4 ou la revendication 5, dans lequel la surface orientée distalement et la surface orientée proximalement présentent des contours non plans.

7. Cathéter selon l'une quelconque des revendications précédentes, dans lequel l'au moins une de la pluralité de pointes (102, 104, 106) est une pointe distale (102) et l'une adjacente de la pluralité de pointes (102, 104, 106) est une pointe médiane (104), la pluralité de pointes (102, 104, 106) comprenant en outre une pointe proximale (106) qui est adjacente à la pointe médiane (104).

8. Cathéter selon l'une quelconque des revendications 4 à 6, dans lequel la surface orientée proximalement comprend une portion s'imbriquant qui s'imbrique avec une portion s'imbriquant de la surface orientée distalement faisant face à la surface orientée proximalement.

9. Cathéter selon la revendication 8, dans lequel l'une des portions s'imbriquant est une saillie (152, 154, 156) et l'autre des portions s'imbriquant est un évidement.
